**Europäisches Patentamt**

**European Patent Office**

**Office européen des brevets**

(19)

(11) Veröffentlichungsnummer: **0 163 212**
**B1**

(12) # EUROPÄISCHE PATENTSCHRIFT

(45) Veröffentlichungstag der Patentschrift:
14.01.87

(51) Int. Cl.⁴: **C 07 C 63/06,** C 07 C 51/487,
C 07 C 51/44

(21) Anmeldenummer: **85106050.9**

(22) Anmeldetag: **17.05.85**

(54) Verfahren zur Reinigung von Benzoesäure.

(30) Priorität: **30.05.84 DE 3420111**

(43) Veröffentlichungstag der Anmeldung:
**04.12.85 Patentblatt 85/49**

(45) Bekanntmachung des Hinweises auf die Patenterteilung:
**14.01.87 Patentblatt 87/3**

(84) Benannte Vertragsstaaten:
**AT DE FR GB IT NL**

(56) Entgegenhaltungen:
**US-A-3 051 746**

(73) Patentinhaber: **BAYER AG, Konzernverwaltung RP Patentabteilung, D-5090 Leverkusen 1 Bayerwerk (DE)**

(72) Erfinder: **Schulte- Huermann, Werner, Dr., Helmut-Macke- Strasse 10, D-4150 Krefeld (DE)**

LIBER, STOCKHOLM 1987

163 212

**Beschreibung**

Die Erfindung betrifft ein Verfahren zur Reinigung von Benzoesäure, die durch katalytische Oxidation von Toluol mit Luft in flüssiger Phase erhalten wurde.

Bei den bekannten Verfahren zur Herstellung von Benzoesäure durch Flüssigphasenoxidation von Toluol mit Luft in Gegenwart von Metallverbindungen als Katalysatoren, z.B. Kobalt- und Manganverbindungen, bei erhöhter Temperatur entsteht Benzoesäure, die u.a. Phthalsäure (vgl. Chem. Zentralbl. 1942, II S. 2642) und größere Mengen an Benzylbenzoat als Verunreinigungen enthält (vgl. Ullmanns Encyclopädie der technischen Chemie, 4. Auflage, Band 8, Seiten 367 bis 369, 1974). Solche Verunreinigungen stören jedoch im allgemeinen die Weiterverarbeitung der Benzoesäure, insbesondere die Weiterverarbeitung der Benzoesäure zu Natriumbenzoat, das in der Regel durch Auflösen einer durch fraktionierte Destillation gereinigten Benzoesäure in Natronlauge erhalten wird.

Ein auf diese Weise hergestelltes Natriumbenzoat ist weder in Ethanol klar löslich und entspricht nicht der Reinheitsanforderung gemäß dem Deutschen Arzneibuch (DAB VII, 689 - 691 (1968)), noch ist die Löslichkeit in Ethylenglykol gegeben, was den Einsatz in Frostschutzmitteln verbietet. Diese Nachteile wurden nach der DE-AS 26 36 489 dadurch beseitigt, daß der Destillation der rohen Benzoesäure kleine Mengen eines Amins, bevorzugt Ammoniak, zugesetzt werden, wodurch die störende Phthalsäure, deren Natriumsalz in Alkohol schwer löslich ist, infolge Amidierung oder Imidierung im Sumpf zurückgehalten und das überschüssige Ammoniak als Benzamid gebunden wird.

Will man, was aus wirtschaftlichen Gründen naheliegend ist, den hauptsächlich aus Benzylbenzoat bestehenden Destillationssumpf der Benzoesäure, der mengenmäßig etwa 10 Gew.-% der eingesetzten Benzoesäure beträgt, destillativ aufarbeiten und verwerten, z.B. durch direkte Chlorierung des aus dem Destillationssumpf erhaltenen Destillats zu Benzoylchlorid (vgl. EP-OS 78 993), so ist das in der DE-AS 26 36 489 beschriebene Reinigungsverfahren nicht geeignet, da das im Destillat enthaltene Benzylbenzoat noch Benzamid enthält, das bei der direkten Chlorierung in Benzonitril umgewandelt wird, so daß ein technisch nicht verwertbares Benzoylchlorid mit einem Benzonitrilgehalt mit bis zu 5 Gew.-% erhalten wird. Die Aufarbeitung des Destillationssumpfes der Benzoesäure war daher bislang technisch nur sinnvoll und wirtschaftlich vertretbar, wenn gleichzeitig auf die Herstellung eines alkohollöslichen Natriumbenzoats, wie oben beschrieben, verzichtet wurde.

Es wurde nun ein Verfahren zur Reinigung von Benzoesäure, die durch katalytische Oxidation von Toluol mit Luft in flüssiger Phase erhalten wurde, gefunden, das dadurch gekennzeichnet ist, daß man die Destillation der rohen Benzoesäure in Gegenwart von aliphatischen Aminen der Formel

$$HNR^1R^2 \qquad ,$$

worin

$R^1$ für Wasserstoff, einen geradkettigen oder verzweigten Hydroxyalkyl- oder Aminoalkylrest mit 1 bis 6 Kohlenstoffatomen und

$R^2$ für einen geradkettigen oder verzweigten Hydroxyalkyl- oder Aminoalkylrest mit 1 bis 6 Kohlenstoffatomen stehen,

und/oder deren Salzen durchführt.

Die Herstellung von Benzoesäure durch katalytische Oxidation von Toluol in flüssiger Phase kann in üblicher Weise erfolgen (vgl. Ullmanns Encyclopädie der technischen Chemie, 4. Auflage, Band 8, Seiten 367 bis 396 (1974)). Von der so erhaltenen Benzoesäure wird üblicherweise das überschüssige Toluol durch Destillation abgetrennt. Eine solchermaßen vorbehandelte Benzoesäure kann dann in das erfindungsgemäße Verfahren für die weitere Reinigung eingesetzt werden. Es ist auch möglich, eine Benzoesäure in das erfindungsgemäße Verfahren einzusetzen, die bereits kristallisiert oder destilliert wurde. Bevorzugt wird eine Benzoesäure in das erfindungsgemäße Verfahren eingesetzt, deren Anteil an Verunreinigungen bis zu 10 Gew.-%, besonders bevorzugt bis zu 8 Gew.-%, beträgt.

Als Amine werden in das erfindungsgemäße Verfahren solche der allgemeinen Formel eingesetzt, deren Reste $R^1$ und $R^2$ einen geradkettigen oder verzweigten Hydroxyalkyl- oder Aminoalkylrest mit 1 bis 6 Kohlenstoffatomen, bevorzugt 2 bis 3 Kohlenstoffatomen besitzen. Beispielsweise seien als geradkettige oder verzweigte Hydroxyalkyl- und Aminoalkylreste genannt: 2-Oxyethyl-, 3-Oxy-n-propyl-, 2-Oxy-iso-propyl-, 4-Oxy-n-butyl-, 3-Oxy-isobutyl-, 2-Oxy-tert.-butyl-, 5-Oxy-n-pentyl-, 6-Oxy-n-hexyl-, 2-Amino-ethyl-, 3-Amino-n-propyl-, 2-Amino-iso-propyl-, 4-Amino-n-butyl-, 3-Amino-isobutyl-, 2-Amin-tert.-butyl-, 5-Amino-n-pentyl- und 6-Amino-n-hexyl-Reste, bevorzugt der 2-Oxyethyl- und der 2-Aminoethyl-Rest.

Als Amine, die man aus Zweckmäßigkeits- oder Kostengründen in das erfindungsgemäße Verfahren einsetzen kann, seien z.B. β-Aminoethanol, Aminopropanol, Amino-n-butanol, Amino-sec.-butanol, Amino-tert.-butanol, Diethanolamin, Dipropanolamin, Ethylendiamin und Diaminoethylen-amin, selbstverständlich auch möglich, neben den freien Aminen deren Salze einzusetzen, soweit sie bei der weiteren Aufarbeitung der Benzoesäure keine Störungen hervorrufen. Bevorzugt sind solche Salze in das erfindungsgemäße Verfahren einsetzbar, deren Säuren gegenüber Benzoesäure leicht flüchtig sind, sich ohne Bildung von Nebenverbindungen zersetzen und keine Korrosion hervorrufen. Als Salze seien beispielsweise die Carbonate, Hydrogencarbonate, Formiate, Acetate, Oxalate und Benzoate, bevorzugt die Carbonate, Hydrogencarbonate und die Benzoate der vorgenannten Amine genannt.

Selbstverständlich ist es für das erfindungsgemäße Verfahren auch möglich, Gemische der Amine und/oder

2

deren Salze einzusetzen. Um das Zudosieren kleinerer Mengen an Aminen zu erleichtern, ist es auch möglich, wäßrige Lösungen der Amine einzusetzen.

Für das erfindungsgemäße Verfahren sind geringe Mengen an Aminen und/oder deren Salze ausreichend. Die jeweils erforderliche Menge richtet sich in erster Linie nach der Qualität der bei der Toluoloxidation entstandenen Benzoesäure und kann nach bekannten analytischen Methoden leicht ermittelt werden. Im allgemeinen ist es jedoch ausreichend, wenn man das Amin oder dessen Salz in Mengen von etwa 0,01 bis 0,001, bevorzugt 0,003 bis 0,006 Mol, bezogen auf 1 Mol Benzoesäure, einsetzt.

Für das erfindungsgemäße Verfahren ist es jedoch ohne Bedeutung, wenn ein Überschuß an Amin eingesetzt wird. Des kann von Vorteil sein wenn bei der chargenweisen Fahrweise einzelne Analysen erspart werden sollen.

Das erfindungsgemäße Verfahren kann in einem Temperaturbereich von etwa 80 bis 250°C, bevorzugt 120 bis 230°C, und bei Drücken von 1 bis 1013 mbar, bevorzugt von 10 bis 600 mbar, durchgeführt werden.

Eine besondere Ausführungsform des erfindungsgemäßen Verfahrens besteht darin, daß die bei der Luftoxidation von Toluol in Gegenwart von Kobalt- Mangan-Katalysatoren gebildete Benzoesäure durch Abdestillieren von nicht verbrauchtem Toluol befreit und in der Wärme durch Rühren mit einem entsprechenden Amin versetzt wird. Die Destillation der Benzoesäure kann in an sich bekannter Weise im Vakuum bei etwa 18 mbar und einer Siedetemperatur von etwa 140°C durchgeführt werden. Nach Abtrennen des Vorlaufs kann das Reinprodukt für die Herstellung von Natriumbenzoat verwendet werden. Das auf diese Weise hergestellte Natriumbenzoat entspricht den Forderungen des Deutschen Arzneibuchs und kann auch, da es in Ethylenglykol klar löslich ist, für den Einsatz in Frostschutzmitteln verwendet werden.

Der Destillationssumpf der Benzoesäure läßt sich ebenfalls in bekannter Weise im Vakuum bei etwa 4 mbar und einer Siedetemperatur von etwa 160 bis 170°C destillativ reinigen. Es wird ein hauptsächlich aus Benzylbenzoat bestehendes Destillat erhalten, welches praktisch frei von Benzamid ist. Wird das auf diese Weise hergestellte Benzylbenzoat der direkten Chlorierung unterworfen, so entsteht ein Benzoylchlorid hoher Reinheit, welches praktisch frei von Benzonitril ist.

Die nachfolgenden Beispiele dienen zur Erläuterung des erfindungsgemäßen Verfahrens. Die für diese Beispiele verwendete Rohbenzoesäure wurde durch Luftoxidation von Toluol in Gegenwart von Kobalt-Mangan-Salzen bei Temperaturen von 160 bis 180°C hergestellt und durch Erhitzen auf 190°C von Toluol befreit.

## Beispiel 1

500 g Rohbenzoesäure mit einem Gehalt von 0,25 Gew.-% Phthalsäureanhydrid wurden mit 0,50 g Ammoniak (als 4,0 g Ammoniumbenzoat, 0,36 Mol/Mol Phthalsäure) vermischt und anschließend im Vakuum über eine 60 cm VA-Drahtnetz-Kolonne destilliert. Bei einer Kopftemperatur von 140°C und einem Vakuum von 18 mbar destillierten 448 g Benzoesäure mit einem Gehalt von 0,09 Gew.-% Phthalsäure über. Das Destillat wurde mit so viel 10 %iger wäßriger Natronlauge versetzt, bis die Lösung einen pH-Wert von 7 hatte. Das aus Nebenverbindungen bestehende Öl wurde abgetrennt, die wäßrige Phase eingedampft und zuletzt im Vakuum getrocknet. 10 g des so erhaltenen Natriumbenzoats wurden unter Rühren in 40 g Ethylenglykol gelöst. Es entstand eine klare Lösung.

Der überwiegend aus Benzylbenzoat bestehende Destillationsrückstand (49 g) wurde über einen Claisenaufsatz destilliert. Die Hauptmenge (42 g) destillierte bei einer Kopftemperatur von 165°C und einem Vakuum von 4 mbar über. Das Destillat enthielt aufgrund der gaschromatographischen Analyse 2,51 Gew.-% Benzamid und einen $N_2$-Gehalt von 0,35 %.

## Beispiele 2 bis 7

Entsprechend der im Beispiel 1 beschriebenen Arbeitsweise wurden die Beispiele 2 bis 7 durchgeführt, und zwar mit Ammoniak wie es Stand der Technik ist, und mit den erfindungsgemäßen bifunktionellen Aminen. Die nachfolgende Tabelle gibt das Ergebnis wieder:

| Beispiel Nr. | Amin/Ammoniakzusatz in Mol/Mol Phthalsäure | Menge in g | Löslichkeit des Natriumbenzoat in Ethylenglykol | Phthalsäuregehalt in % | Destillat d. Rückstands Benzamidgehalt % | Destillat d. Rückstands $N_2$-Gehalt % |
|---|---|---|---|---|---|---|
| 2 | 5,0 Ammoniak | 0,71 | klar | 0,02 | 5,46 | 0,76 |
| 3 | 2,0 Ammoniak | 0,28 | leicht trübe | 0,11 | 2,01 | 0,28 |
| 4 | 2,0 Ethanolamin | 1,04 | klar | 0,08 | 0,01 | 0,02 |
| 5 | 3,6 Ethanolamin | 1,86 | klar | < 0,01 | 0,03 | 0,09 |
| 6 | 2,0 Propanolamin | 1,21 | klar | < 0,01 | 0,05 | 0,07 |
| 7 | 1,5 Ethylendiamin | 0,73 | klar | < 0,01 | 0,06 | 0,03 |

## Patentansprüche

1. Verfahren zur Reinigung von Benzoesäure, die durch katalytische Oxidation von Toluol mit Luft in flüssiger Phase erhalten wurde, dadurch gekennzeichnet, daß man die Destillation der rohen Benzoesäure in Gegenwart vor aliphatischen Aminen der Formel

4

$HNR^1R^2$,

worin

$R^1$ für Wasserstoff, einen geradkettigen oder verzweigten Hydroxyalkyl- oder Aminoalkylrest mit 1 bis 6 Kohlenstoffatomen und

$R^2$ für einen geradkettigen oder verzweigten Hydroxyalkyl- oder Aminoalkylrest mit 1 bis 6 Kohlenstoffatomen stehen,

und/oder deren Salzen durchführt.

2. Verfahren nach Anspruch 1, dadurch gekennzeichnet, daß man die Destillation in Gegenwart von β-Aminoethanol, Aminopropanol, Amino-n-butanol, Aminosec.-butanol, Amino-tert.-butanol, Diethanolamin, Dipropanolamin, Ethylendiamin und/oder Diaminoethylen-amin durchführt.

3. Verfahren nach Ansprüchen 1 und 2, dadurch gekennzeichnet, daß man die Destillation in Gegenwart von β-Aminoethanol und/oder Ethylendiamin durchführt.

4. Verfahren nach Ansprüchen 1 bis 3, dadurch gekennzeichnet, daß man die Amine in Mengen von 0,001 bis 0,01 Mol, bezogen auf 1 Mol Benzoesäure, einsetzt.

5. Verfahren nach Ansprüchen 1 bis 4, dadurch gekennzeichnet, daß man die Amine in Mengen von 0,003 bis 0,006 Mol, bezogen auf 1 Mol Benzoesäure, einsetzt.

6. Verfahren nach Ansprüchen 1 bis 5, dadurch gekennzeichnet, daß man die Destillation bei Temperaturen von 80 bis 250°C durchführt.

## Claims

1. Process for the purification of benzoic acid which has been obtained by catalytic oxidation of toluene with air in the liquid phase, characterised in that the distillation of the crude benzoic acid is carried out in the presence of aliphatic amines of the formula $HNR^1R^2$,

wherein

$R^1$ represents hydrogen or a straight-chain or branched hydroxyalkyl or aminoalkyl radical with 1 to 6 carbon atoms and

$R^2$ represents a straight-chain or branched hydroxyalkyl or aminoalkyl radical with 1 to 6 carbon atoms,

and/or salts thereof.

2. Process according to Claim 1, characterised in that the distillation is carried out in the presence of β-aminoethanol, aminopropanol, amino-n-butanol, aminosec.-butanol, amino-tert.-butanol, diethanolamine, dipropanolamine, ethylenediamine and/or diaminoethylene-amine.

3. Process according to Claims 1 and 2, characterised in that the distillation is carried out in the presence of β-aminoethanol and/or ethylenediamine.

4. Process according to Claims 1 to 3, characterised in that the amines are employed in amounts of 0.001 to 0.01 mol, relative to 1 mol of benzoic acid.

5. Process according to Claims 1 to 4, characterised in that the amines are employed in amounts of 0.003 to 0.006 mol, relative to 1 mol of benzoic acid.

6. Process according to Claims 1 to 5, characterised in that the distillation is carried out at temperatures of 80 to 250°C.

## Revendications

Procédé de purification d'acide benzoique qui a été obtenu par oxydation catalytique en phase liquide du toluéne par l'air, caractérisé en ce qu'on conduit la distillation de l'acide benzoique brut en présence d'amines aliphatiques de formule

$HNR^1R^2$

dans laquelle

$R^1$ représente l'hydrogène, un reste hydroxyalkyle ou aminoalkyle à chaine droîte ou à chaîne ramifiée ayant 1 à 6 atomes de carbone et

$R^2$ représente un reste hydroxyalkyle ou aminoalkyle à chaîne droite ou à chaîne ramifiée ayant 1 à 6 atomes de carbone,

et/ou de leurs sels.

2. Procédé suivant la revendication 1, caractérisé en ce qu'on conduit la distillation en présence de β-aminoéthanol d'aminopropanol, d'aminon-butanol, d'amino-sec. -butanol, d'amino-tertio-butanol, de diéthanolamine, de dipropanolamine, d' éthylènediamine et/ou de diaminoéthylène-amine.

3. Procédé suivant les revendications 1 et 2, caractérisé en ce qu'on conduit la distillation en présence de β-aminoéthanol et/ou d' éthylènediamine.

4. Procédé suivant les revendications 1 à 3, caractérisé en ce qu'on utilise les amines en quantités de 0,001 à 0,01 mole, pour 1 mole d'acide benzoïque.

5. Procédé suivant les revendications 1 à 4, caractérisé en ce qu'on utilise les amines en quantités de 0,003 à 0,006 mole pour 1 mole d'acide benzoique.

6. Procédé suivant les revendications 1 à 5, caractérisé en ce qu'on conduit la distillation à des températures de 80 à 250°C.